# EUROPÄISCHE PATENTSCHRIFT

(11) **EP 1 059 875 B1**
(45) Veröffentlichungstag und Bekanntmachung des Hinweises auf die Patenterteilung: **17.10.2001**
(21) Anmeldenummer: 98916839.8
(22) Anmeldetag: 04.03.1998
(51) Int. Cl.: A61B 5/02, G06F 19/00

(54) **AUSWERTESYSTEM ZUR GEWINNUNG DIAGNOSTISCHER INFORMATIONEN AUS SIGNALEN UND DATEN MEDIZINISCHER SENSORSYSTEME**
EVALUATION SYSTEM FOR OBTAINING DIAGNOSTIC INFORMATION FROM THE SIGNALS AND DATA OF MEDICAL SENSOR SYSTEMS
SYSTEME D'EVALUATION POUR L'ACQUISITION D'INFORMATIONS DIAGNOSTIQUES A PARTIR DE SIGNAUX ET DE DONNEES DE SYSTEMES DETECTEURS A USAGE MEDICAL

(43) Veröffentlichungstag der Anmeldung: 20.12.2000
(73) Patentinhaber: Bundesr. Deutschland, vertr. d. d. Bundesministerium für Wirtschaft und Techn., vert. d. den Präs. der Physi.Tech. Bundesansta, 38116 Braunschweig (DE)
(72) Erfinder: Bousseljot, Ralf, 15711 Königs Wusterhausen (DE); Kreiseler, Dieter, 10551 Berlin (DE)
(74) Vertreter: Lins, Edgar, Dipl.-Phys. Dr.jur.
(86) Internationale Anmeldenummer: DE9800625
(87) Internationale Veröffentlichungsnummer: WO9944498

(56) Entgegenhaltungen:
- WO-A-92/05831
- DE-A- 19 638 738
- US-A- 5 277 188
- US-A- 5 282 474
- US-A- 5 586 024
- US-A- 5 735 286

## Beschreibung

### Anwendungsgebiet der Erfindung:

Die Erfindung betrifft ein Auswertesystem zur Gewinnung diagnostischer Informationen aus Signalen und Daten medizinischer Sensorsysteme mit Hilfe von Meßdaten- und Patienten-Datenbanken.

### Charakteristik bekannter technischer Lösungen:

In zunehmendem Maße werden in der medizinischen Diagnostik Systeme eingesetzt, die aus vom Patienten abgeleiteten Signalen und Daten diagnostische Informationen gewinnen. Zu dieser Gruppe von Systemen gehören auch die automatisch auswertenden Elektrokardiografen (EKG-Geräte).

EKG-Auswertesysteme, z.B. nach US 5,022,404, erfassen ein oder mehrere Elektrodenpotentiale von am Patienten befestigten Elektroden, filtern und digitalisieren diese. Anschließend werden diese Signale über einen Multiplexer an einen im EKG-Auswertesystem vorhandenen Mikrocomputer mit CPU, Arbeitsspeicher usw. zugeführt. Dieser bereitet die gemessenen Signale auf, z.B. durch Entfernen der Grundliniendrift nach DE 41 06 856, US 5,357,969 oder die Entfernung von Muskelartefakten aus dem EKG nach US 5,259,387. Außerdem berechnet er die für die medizinische Bewertung des EKG erforderlichen medizinischen Ableitungen nach Wilson, Goldberger, Einthoven und/oder die orthogonalen Ableitungen nach Frank. Im einfachsten Fall werden diese medizinischen Ableitungen entweder auf Papierstreifen und/oder elektronischen Displays, z.B. in US 5,022,404 auf LCD-Displays, dargestellt und vom auswertenden Arzt bewertet. Intelligentere, sogenannte auswertende Elektrokardiografen, verwenden den im Gerät vorhandenen Mikrocomputer außer zur Signalaufbereitung und Anzeige auch zur Signalauswertung, Signalvermessung und ggfs. zur Ausgabe von diagnostischen Hinweisen, wie z.B. in US 5,029,082.

Die Signalvermessung und Auswertung erfolgt, wie noch in nachfolgend näher erläuterten Patentschriften beschrieben wird, in der Regel so, daß aus den berechneten medizinischen Ableitungen eine Anzahl, für die kardiologische Begutachtung des EKG wichtiger, einzelner Signalparameter hinsichtlich Zeitdauer und Amplitude bzw. daraus abgeleiteten Kriterien ermittelt werden. Problematisch bei dieser Ermittlung einzelner Signalkenngrößen sind die unterschiedlichen Herangehensweisen, wie z.B. bei der exakten Bestimmung der Nullinie des EGK zur Bestimmung des Anfangspunktes der P-Welle und der daraus folgenden Ermittlung der Dauer der P-Welle, die je nach Qualität des eingesetzten Verfahrens durchaus wesentlich abweichende Ergebnisse liefern. Die Patentschriften sind unter anderem DE 43 10 412 (Auswertung des ST-Segments bzw. der T-Welle), DE 39 27 709 (Auswertung der ST-Strecke), US 5,159,932 (Filterung des EKG, QRS-Findung, Mittelung) oder US 5,020,540 (Analyse der Frequenzstruktur des QRST-Komplexes, Waveform-Template). Weitere relevante Patentschriften beinhalten die Ermittlung einzelner Kenngrößen des EKG, bzw. dienen der Erkennung begrenzter diagnostischer Aussagen z.B. in US 4,930,075 (Auswertung des ST-Segments zur Feststellung von Ischämien), US 5,025,794 (Methode der bidirektionalen Filterung zur Erkennung von Spätpotentialen), US 5,355,891 (Automatische Signalmittelung durch Schlagtriggerung zur Erkennung von Spätpotentialen), US 5,341,811 (HP-Filterung von mindestens zwei Kanälen, gewichtete Mittelung, Einsatz adaptiver Filter zur Gleichtaktunterdrückung, Spätpotentialerkennung) oder DE 43 04 269 (Auswertung der ST-Strecke zur Bewertung akuter ischämischer Schädigungen).

Die ermittelten Signalkenngrößen werden direkt zusammen mit dem Signalverlauf des EKG auf dem Papierstreifen ausgedruckt oder angezeigt. Zur Ausgabe diagnostischer Hinweise werden in einem mehr oder weniger komplizierten und verzweigten Entscheidungsbaum die einzelnen ermittelten Signalkenngrößen miteinander zu sinnvollen diagnostischen Hinweisen verknüpft. Dies erfolgt beispielsweise durch die vielen Computer-EKG-Geräten zugrunde liegenden Programme. Solche Entscheidungsbäume können beispielsweise folgende Form haben: "Wenn Parameter 1 in Verbindung mit Parameter 3 und/oder Parameter 4 auftritt und in der medizinischen Ableitung a gleichzeitig Bedingung 1 wirksam ist, kann daraus auf die diagnostische Aussage xyz geschlossen werden." In dieser Weise kann für jede bekannte Diagnose ein Entscheidungsbaum auf der Grundlage einzelner, aus dem EKG in seinen Ableitungen ermittelter Signalkenngrößen aufgebaut werden. Dieses Verfahren ist aufgrund der Vielzahl der Einflußgrößen und Parameter außerordentlich aufwendig und setzt umfangreiche kardiologische Kenntnisse bzw. Erfahrungen voraus. Änderungen oder Verbesserungen der Verfahren zur Ermittlung einzelner Parameter, Beeinflussung von empirisch bestimmten Schwellwerten oder neue medizinische Erkenntnisse erfordern teilweise aufwendige Programmänderungen und Funktionstests und sind daher mit hohen Kosten verbunden, bzw. erfordern neue EKG-Geräte mit den überarbeiteten Programmen. Das Patent US 5,355,892 beschreibt daher ein EKG-System mit portablen Speichermedien (Diskettenlaufwerk) zur Speicherung sowohl von EKG und Patienteninformationen, z.B. für Krankenhaus-Informationssysteme, als auch zum Nachladen oder Updaten von Algorithmen zur EKG-Auswertung.

Eine neuere Gruppe von EKG-Geräten bzw. Verfahren versuchen eine begrenzte diagnostische Information aus dem EKG-Signal mittels lernfähiger neuronaler Netze zu gewinnen, wie z.B. DE 43 07 545 (Vielkanalmessung elektrischer und/oder magnetischer Feldgrößen mindestens während eines Teils des Herzzyklus, Auswertung mit einem lernfähigen Klassifikator (neuronales Netz zur Klassifizierung und Lokalisierung von Ischämien und/oder Infarkten) oder US 5,280,792 (Arrythmia-Klassifizierung mit Hilfe der Kombination von Zeitanalyse und Mustervergleich sowie neuronalen Netzen)). Problematisch bei diesen Verfahren ist sowohl der hohe Lernaufwand beim Training der neuronalen Netze als auch die Tatsache, daß neuronale Netze sich selbst im Stadium der angewandten Grundlagenforschung befinden. Eine vollständige Analyse eines EKG mit dem Ziel der Ableitung komplexer diagnostischer Aussagen erfordert aufrund der Komplexität eines EKG eine sehr hohe Anzahl von Neuronen bzw. damit verbunden eine sehr hohe Rechenleistung.

Eine weitere Gruppe von EKG-Geräten bzw. Verfahren dient der Analyse und Diagnostizierung von Rhythmusstörungen, z.B. für Herzschrittmacher oder die direkte Ansteuerung von Defibrillatoren. Dazu zählen z.B. die Patentschriften DE 32 09 850 (Klassifizierung von Rhythmusstörungen, Auswertung durch Vergleich des vollständigen Verlaufs des EKG mit zuvor in einer Lernphase erfaßten oder berechneten EKG-Verläufen des untersuchten Patienten, vollständige Abspeicherung eines Beispiels des EKG-Verlaufs für jede Klassen von Rhythmusstörungen des untersuchten Patienten), US 5,240,009 (Erkennung von Rhythmusstörungen durch Vergleich gemittelter und gespeicherter Wellenformkomplexe mit aktuellen des selben Patienten), US 5,217,021 (Korrelation von befundfreien Teilen des eigenen EKG mit Bereichen des EKG mit Rhythmusstörungen, Kompression und Speicherung der befundfreien EKG-Bereiche, Alarmkriterium ist die Schwellwertüberschreitung der KKF) oder DE 43 20 519 (Messung und Vergleich von 3 Herzschlagperioden und danach von mindestens 50 Herzschlagkurven, Diagnose von Herzrhythmusstörungen). All diesen Verfahren ist gemeinsam, daß sie jeweils nur bestimmte Diagnosegruppen bzw. definierte Einzelmerkmale des EKG untersuchen.

### Ziel der Erfindung:

Die Erfindung ermöglicht es, von aufgenommenen Signalen und Daten elektrischer und/oder magnetischer medizinischer Meßsysteme diagnostische Aussagen mit hoher Zuverlässigkeit abzuleiten, ohne die Meßdaten zunächst auf mehr oder weniger komplexe, vom Erkenntnisfortschritt abhängende Einzelmerkmale zu reduzieren und diese Einzelmerkmale anschließend mit Hilfe von schwer modifizierbaren Entscheidungsbäumen oder neuronalen Netzen zu einer diagnostischen Aussage zu verknüpfen.

### Darstellung des Wesens der Erfindung:

Der Erfindung liegt die Aufgabe zugrunde, die Gewinnung diagnostischer Informationen aus den Meßdaten medizinischer Meßsysteme, wie z.B. EKG-Systemen weitgehend unabhängig von der mehr oder weniger komplexen und sich mit dem Erkenntnisfortschritt laufend ändernden Auswertung von Einzelmerkmalen und darauf aufbauenden Entscheidungsbäumen bzw. dem umfangreichen Trainingsprozeß neuronaler Netzwerke zu machen. Erfindungsgemäß wird die Aufgabe dadurch gelöst, daß die von einem Patienten aufgenommenen Meßdaten eines oder mehrerer Sensorkanäle einer Messung durch einen Vergleicher mit allen oder mit einem Teil der in einer oder mehreren Meßdatenbanken gespeicherten Meßdaten vergleichbarer Sensorkanäle von Referenzmessungen in der Weise verglichen werden, daß aus den Meßdatenbanken diejenigen Referenzmessungen ausgewählt werden, die zu der vom Patienten aufgenommenen Messung sowohl hinsichtlich der Meßdaten vergleichbarer Sensorkanäle die größte Ähnlichkeit als auch eine größtmögliche Anzahl der in den Meßdaten am meisten übereinstimmenden und einander entsprechenden Sensorkanäle besitzen und über die mit Hilfe der zu den Referenzmessungen der Meßdatenbank gehörigen technischen, medizinischen, diagnostischen und personengebundenen Informationen und deren Vergleich mit den zu dem Patienten gehörenden Informationen auf eine den Patienten mit einer bestimmten Wahrscheinlichkeit zutreffenden diagnostischen Aussage geschlossen werden kann.

### Ausführungsbeispiel:

Die Erfindung soll nachfolgend an zwei Ausführungsbeispielen näher erläutert werden. In den dazugehörigen Zeichnungen zeigen
- Fig. 1:: Prinzipdarstellung eines lokalen EKG-Aufnahmesystems mit einer Datenbank auf CD-ROM
- Fig. 2:: Schematische Darstellung der Funktion eines Beispiels des Vergleichers zur Ableitung diagnostischer Informationen aus den aufbereiteten Meßdaten
- Fig. 3:: Darstellung des Auswerteergebnisses eines EKG gegenüber 370 EKG einer Signaldatenbank (Herzinfarkte)
- Fig. 4:: Prinzipdarstellung eines EKG-Aufnahmesystems mit einer netzwerkbasierten SQL-Datenbank und netzwerkbasierter Auswertung.

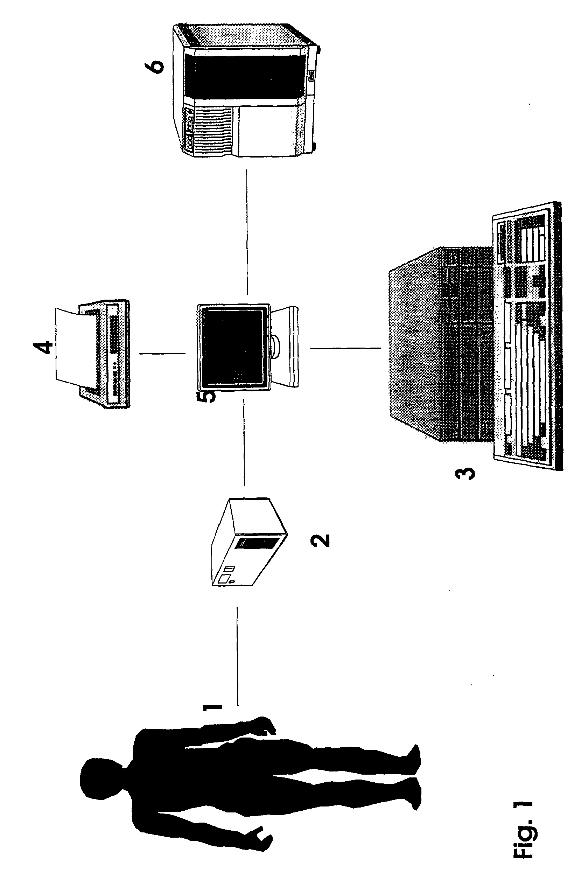

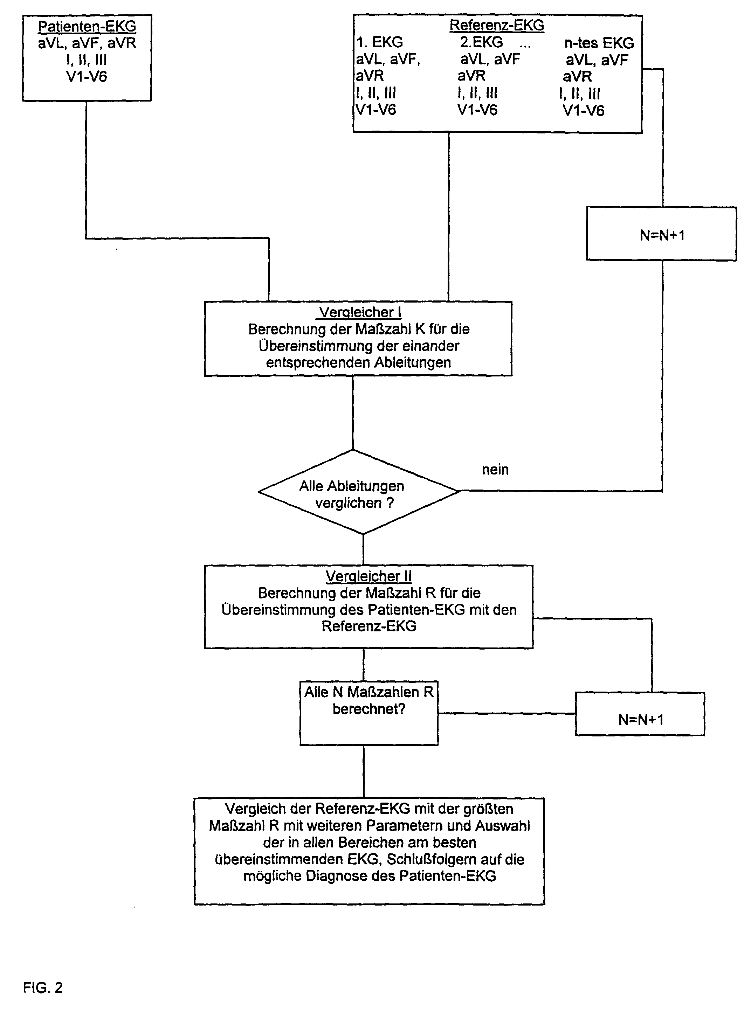

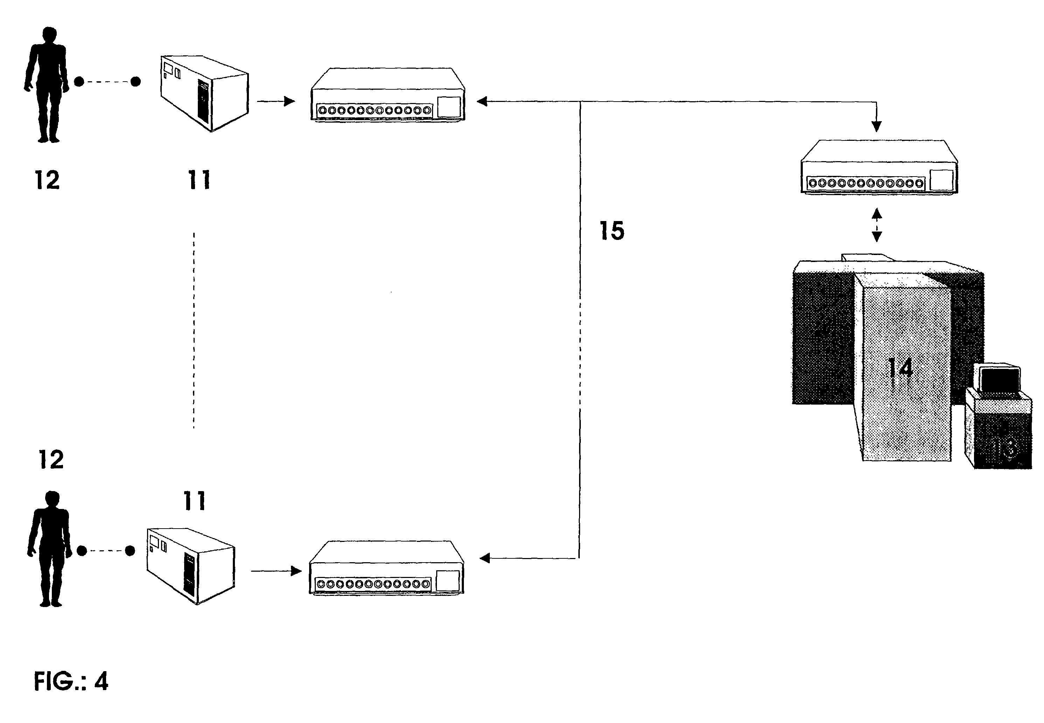

In Fig. 1 wird die Erfindung am Beispiel eines lokalen EKG-Aufnahme- und Auswertesystem näher erläutert. Über in üblicher Weise am Patienten befestigte Elektroden (1) werden die elektrischen Potentiale des Herzens des Patienten erfaßt und von einem Meßwertaufbereitungssystem (2) verstärkt, gefiltert und digitalisiert. Aus diesen aufbereiteten Meßsignalen werden die medizinischen Ableitungen, z.B. nach den 12-Kanal-Standardableitungen durch den im EKG-Aufnahme- und Auswertesystem befindlichen Mikrocomputer (3) berechnet und in ihrem Zusammenhang als EKG-Messung gespeichert und ggfs. über einen Drucker (4) ausgegeben oder auf einem Monitor (5) dargestellt. Auf einer oder mehreren CD-ROM, die sich in einem an den Mikrocomputer (3) angeschlossenen CD-ROM-System (6) befinden, ist eine Datenbank gespeichert, die zu einem früheren Zeiptunkt aufgenommene EKG-Messungen sowie weitere Informationen zu diesen EKG-Messungen enthält. Dabei ist es unerheblich, ob die Datenbank sich auf CD-ROM oder anderen geeigneten elektronischen Massenspeichern befindet. Die EKG-Signale der in der Datenbank gespeicherten Referenzmessungen liegen im vorliegenden Ausführungsbeispiel ebenfalls als 12-Kanal-Standardableitungen vor. Dabei entspricht die Bandbreite der in der Datenbank enthaltenen, digitalisierten EKG-Signale entsprechend dem Nyqiust-Theorem maximal der halben Abtastrate der mit dem EKG-Aufnahme- und Auswertesystem aufgenommenen EKG-Signale. Zu jedem, in der Datenbank enthaltenen Patienten können im vorliegenden Beispiel mehrere EKG-Aufnahmen in bestimmten zeitlichen Abständen gespeichert sein. Zu den weiteren in der Datenbank gespeicherten Informationen gehören patientenbezogene Informationen, Informationen zur Anamnese, Untersuchungsbefunde, Laborwerte, medizinische Diagnosen, Informationen über die Art und Weise der Behandlung des Patienten, verabreichte Medikamente usw. Über ein im Ausführungsbeispiel durch ein Computerprogramm im Mikrocomputer (3) gebildeten Vergleicher (s. Fig. 2) werden erfindungsgemäß die von einem Patienten aufgenommenen EKG-Ableitungen einer Messung durch den Vergleicher mit in einer oder mehreren Meßdatenbanken gespeicherten EKG-Ableitungen von Referenzmessungen in der Weise verglichen, daß aus der Meßdatenbank diejenigen Referenzmessungen ausgewählt werden, die zu der vom Patienten aufgenommenen Messung sowohl hinsichtlich der Meßdaten vergleichbarer Sensorkanäle die größte Ähnlichkeit als auch eine größtmögliche Anzahl der in den Meßdaten übereinstimmenden und einander entsprechenden Sensorkanäle besitzen und über die zu den Referenzmessungen der Meßdatenbank gehörigen medizinischen, diagnostischen und personengebundenen Informationen auf eine den Patienten mit einer bestimmten Wahrscheinlichkeit zutreffenen diagnostische Aussage geschlossen werden kann. Der Vergleicher kann zur Einsparung von Rechenzeit auch ganz oder teilweise als Hardwareschaltung ausgeführt sein. Der prinzipielle Ablauf der Auswertung ist in der Fig. 2 näher ausgeführt. Im Beispiel werden dazu in einem ersten Schritt die einzelnen medizinischen Ableitungen aVL, aVR, aVF, V1-V6, I, II, III der 12-Kanal-Standard-Ableitung mit den vergleichbaren, in der Datenbank abgespeicherten, medizinischen Ableitungen im Hinblick auf die Übereinstimmung ihrer Signalmuster verglichen. Das bedeutet z.B. die Ableitng V1 des Patienten wird mit denen in der Datenbank enthaltenen Ableitungen V1 verglichen und eine Maßzahl K₁₁ bis K₁ₙ für den Grad der Übereinstimmung der Ableitung V1 mit den Ableitungen V1ₙ der n Referenz-EKG der Datenbank berechnet. Dasselbe wird für jede medizinische Ableitung wiederholt, so daß im Beispiel für jedes verglichene EKG der Datenbank 12 Maßzahlen K₁ₙ bis K₁₂ₙ (Bei Berücksichtigung von 12 Ableitungen) für den Grad der Übereinstimmung der jeweiligen vergleichbaren Ableitungen vorliegen.

Diese 12 Maßzahlen für jedes EKG werden in einem zweiten Schritt wiederum in geeigneter Weise zu einer Maßzahl R zusammengefaßt, die eine Übereinstimmung des aufgenommenen mit jedem in der Datenbank gespeicherten EKG in seinen Ableitungen beschreibt.

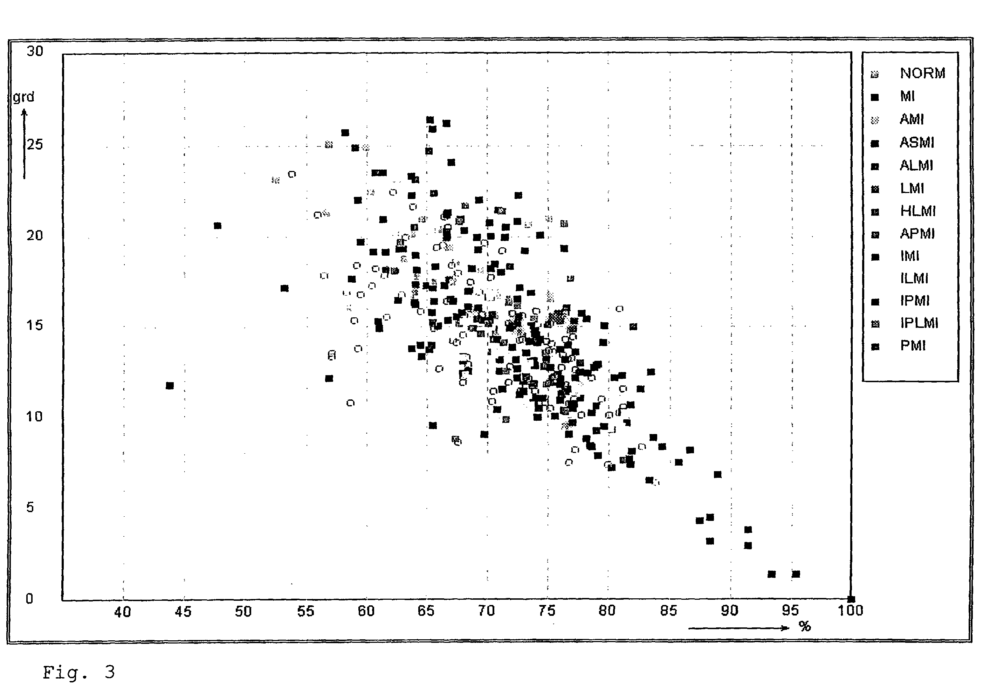

Diejenigen EKG mit der größten Übereinstimmung in allen ihren Ableitungen werden ausgewählt, die das höchste Maß an Übereinstimmung ihrer Signalmuster aufweisen. Aus dieser Anzahl von EKG werden in einem dritten Schritt erfindungsgemäß diejenigen EKG ausgewählt, bei denen hinsichtlich weiterer in der Datenbank enthaltenen Informationen eine größtmögliche Übereinstimmung besteht. Ein mögliches Ergebnis dieser Auswahl für Herzinfarkte zeigt Fig. 3. In diesem Bild ist in einer zweidimensionalen Darstellung der Grad der Übereinstimmung des unbekannten Patienten-EKG mit den bekannten Datenbank-EKG grafisch dargestellt. Je näher ein Datenbank-EKG in der rechten unteren Ecke plaziert ist, desto größer ist seine Übereinstimmung mit dem unbekannten EKG des Patienten. Eine weitergehende Differenzierung der Auswahl bei mehreren mit dem Patienten-EKG gut übereinstimmenden Datenbank-EKG kann durch Zuordnung der in der Datenbank enthaltenen diagnostischen Information zu denen des Patienten gewonnen werden.

Ein wesentlicher Vorteil der erfindungsgemäßen Lösung ist die Möglichkeit, durch einfache Erweiterung der Zahl der in der Datenbank enthaltenen EKG und der dazugehörigen Referenzinformationen die Erkennungssicherheit von EKG-Analysesystemen zu verbessern, indem die natürliche biologische Variabilität repräsentativer widergespiegelt wird und damit die Wahrscheinlichkeit steigt, ein oder mehrere dem Patienten-EKG ähnliche EKG in der Datenbank zu finden.

In Fig. 4 wird eine andere mögliche Ausführungsform der Erfindung beschrieben. Über an ein Netzwerk angeschlossene EKG-Sensorsysteme (11) werden in klassischer Weise an bestimmten Punkten des Oberkörpers der Patienten (12) die elektrischen Potentiale des Herzfeldes gemessen, gefiltert, digitalisiert und stellen in ihrer Gesamtheit die Messung dar. Eine Datenbank (13) mit Meßsignalen vergleichbarer EKG-Messungen zusammen mit weiteren zu den Meßsignalen gehörigen Informationen, sogenannte Referenzmessungen, befinden sich in diesem Ausführungsbeispiel auf einem für diesen Zweck an einem zentralen Ort eingerichteten, leistungsfähigem Computersystem (14), welches ebenfalls an ein Netzwerk angeschlossen ist. Sowohl das EKG-Sensorsystem als auch das die Datenbank enthaltende Computersystem (14) sind über ein lokales oder weltweites Netzwerk (15) ausreichender Übertragungskapazität miteinander verbunden. Dieses Netzwerk (15) kann z.B. durch das LAN in einem Krankenhaus, aber auch durch Telefonleitungen, ISDN-Wählleitungen oder das Internet gebildet werden.

Durch das EKG-Meßwertaufbereitungssystem (11) werden die Meßdaten, zusammen mit anderen, den Patienten (12) betreffenden Angaben, in einem ersten Schritt über das Netzwerk (15) zu dem Computersystem (14) mit der angeschlossenen Datenbank (13) übermittelt. Durch das Computersystem (14) werden, wie bereits beispielsweise in Fig. 2 näher erläutert, diejenigen EKG der Datenbank (13) ausgewählt, die sowohl in ihren einzelnen medizinischen Ableitungen als auch in der Gesamtheit ihrer Messung eine größtmögliche Übereinstimmung aufweisen, und für die hinsichtlich weiterer Angaben zwischen Patient und Datenbank größtmögliche Übereinstimmung besteht. Im Anschluß an die Auswertung durch das Computersystem (14) werden die Ergebnisse des Vergleichs über das Netzwerk (15) an das EKG-Meßwertaufbereitungssystem (11) zurück übermittelt und am Ort des EKG-Sensorsystems angezeigt oder in geeigneter Form ausgegeben oder direkt in entsprechende Krankenhausinformationssysteme eingespeist.

Vorteile dieser Lösung bestehen in der Möglichkeit, an einem zentralen Ort ein wesentlich leistungsfähigeres Computersystem (14) mit einer wesentlich größeren und komplexeren Datenbank aufzubauen, als dies in einem dezentralen, klassisch auswertenden EKG-Gerät möglich wäre. Dies führt zu einer exakteren Aussage über das zu bewertende EKG, da diese nur von der Qualität der Datenbank, d.h. der Anzahl der gespeicherten EKG-Messungen und deren Übereinstimmung mit der möglichen biologischen Vielfalt der kardiologischen Befunde abhängt. Weiterhin können bei entsprechender Leistungsfähigkeit sowohl des Netzwerkes (15), des zentralen Computers (14) sowie der Datenbank (13) gleichzeitig mehrere EKG-Sensorsysteme bedient werden. Außerdem lassen sich auf diese Weise die Kosten für das EKG-Aufnahmesystem reduzieren, da dieses keine eigene diagnostische Auswertung bzw. keine eigene Datenbank beinhalten muß.

Wird ein solches System beispielsweise in einer kardiologischen Klinik mit sehr hohem EKG-Aufkommen eingerichtet, so können zu einem geeigneten Zeitpunkt, beispielsweise nach Abschluß der Behandlung eines Patienten, dessen EKG-Signale und anderen personenbezogenen Informationen der Datenbank hinzugefügt und somit die Basis der Datenbank erweitert werden. Damit entsteht eine immer besser die biologische Vielfalt repräsentierende Wissensbasis.

## Patentansprüche

1. Auswertesystem zur Gewinnung diagnostischer Informationen aus Signalen und Daten medizinischer Sensorsysteme, das gleichzeitig mit einem oder mehreren Sensorkanälen die bilogischen Aktivitäten eines Patienten über einen bestimmten Zeitraum erfaßt, die gemessenen Daten jedes Sensorkanals aufbereitet und in ihrer Gesamtheit als Meßdaten einer Messung zur Ableitung diagnostischer Informationen bereitstellt, **gekennzeichnet dadurch**, **daß** die von einem Patienten aufgenommenen Meßdaten eines oder mehrerer Sensorkanäle einer Messung **durch** einen Vergleicher mit allen oder mit einem Teil der in einer oder mehreren Meßdatenbanken gespeicherten Meßdaten vergleichbarer Sensorkanäle von Referenzmessungen in der Weise verglichen werden, **daß** aus den Meßdatenbanken diejenigen Referenzmessungen ausgewählt werden, die zu der vom Patienten aufgenommenen Messung sowohl hinsichtlich der Meßdaten vergleichbarer Sensorkanäle die größte Ähnlichkeit als auch eine größtmögliche Anzahl der in den Meßdaten am meisten übereinstimmenden und einander entsprechenden Sensorkanäle besitzen und über die mit Hilfe der zu den Referenzmessungen der Meßdatenbank gehörigen technischen, medizinischen, diagnostischen und personengebundenen Informationen und deren Vergleich mit den zu dem Patienten gehörenden Informationen auf eine den Patienten mit einer bestimmten Wahrscheinlichkeit zutreffenen diagnostische Aussage geschlossen werden kann.

2. Auswertesystem zur Gewinnung diagnostischer Informationen aus Signalen und Daten medizinischer Sensorsysteme nach Punkt 1, **gekennzeichnet dadurch**, **daß** die gemessenen und in ihrer Gesamtheit als Messung erfaßten Meßdaten eines oder mehrerer Sensorkanäle sowohl eine kontinuierliche Folge von Meßwerten über einen bestimmten Zeitraum als auch ein oder mehrere, zeitlich begrenzte Ausschnitte aus dieser kontinuierlichen Folge von Meßwerten darstellen können.

3. Auswertesystem zur Gewinnung diagnostischer Informationen aus Signalen und Daten medizinischer Sensorsysteme nach Punkt 2, **gekennzeichnet dadurch**, **daß** der Vergleicher sowohl als ein Computerprogramm, eine elektronische Schaltung oder eine Kombination aus beidem ausgeführt werden kann.

4. Auswertesystem zur Gewinnung diagnostischer Informationen aus Signalen und Daten medizinischer Sensorsysteme nach Punkt 1 bis 3, **gekennzeichnet dadurch**, **daß** neben dem Vergleich der Meßdaten des Patienten und der Meßdatenbank weitere vom Patienten bekannte und in Meßdatenbank gespeicherte personenbezogene Informationen zu Erhöhung der Wahrscheinlichkeit der den Patienten betreffenden diagnostischen Aussage herangezogen werden können.

5. Auswertesystem zur Gewinnung diagnostischer Informationen aus Signalen und Daten medizinischer Sensorsysteme nach Punkt 1 bis 4, **gekennzeichnet dadurch**, **daß** die Meßdatenbank sowohl Meßdaten als auch von den Meßdaten abgeleitete Informationen von Messungen sowohl gesunder Personen als auch kranker Personen enthalten kann, die die biologische Vielfalt der medizinischen Krankheitsbilder und Patienten möglichst gut widerspiegelt.

6. Auswertesystem zur Gewinnung diagnostischer Informationen aus Signalen und Daten medizinischer Sensorsysteme nach Punkt 1 bis 5, **gekennzeichnet dadurch**, **daß** die Meßdatenbank **durch** neue Referenzdatensätze erweitert werden kann.

7. Auswertesystem zur Gewinnung diagnostischer Informationen aus Signalen und Daten medizinischer Sensorsysteme nach Punkt 1 bis 6, **gekennzeichnet dadurch**, **daß** die Meßdatenbank entweder auf einem elektronischen Speichermedium unmittelbar im oder am Auswertesystem gespeichert ist oder über ein lokales oder weltweites Datennetz verfügbar ist und zwischen dem Aufnahmesystem, dem Vergleicher und der Referenzdatenbank eine räumliche Trennung bestehen kann.

8. Auswertesystem zur Gewinnung diagnostischer Informationen aus Signalen und Daten medizinischer Sensorsysteme nach Punkt 1 bis 7, **gekennzeichnet dadurch**, **daß** die Signale und Daten von verschiedenen bioelektrischen und/oder biomagnetischen und/oder anderen biologischen Sensorsystemen stammen können.

9. Auswertesystem zur Gewinnung diagnostischer Informationen aus Signalen und Daten medizinischer Sensorsysteme nach Punkt 1 bis 8, **gekennzeichnet dadurch**, **daß** die von einem Patienten aufgenommenen Signale Elektrodenpotentiale eines EKG-Aufnahmesystems und/oder Signale von magnetischen Sensoren eines ein- oder multikanaligen kardiologischen Aufnahmesystems sein können.

## Claims

1. An evaluating system for obtaining diagnostic information from signals and data of medical sensor systems, which with one or more sensor channels simultaneously acquires the biological activities of a patient over a certain period, edits the measured data of each sensor channel and makes them available in their totality as measured data of a measurement for the purpose of deriving diagnostic information, **characterised in that** the measured data of one or more sensor channels of a measurement that have been recorded from a patient are compared by a comparator with all or with some of the measured data, stored in one or more measurement databases, of comparable sensor channels of reference measurements in such a way that those reference measurements are selected from the measurement databases which in relation to the measurement recorded from the patient possess both the greatest similarity with regard to the measured data of comparable sensor channels and a largest possible number of sensor channels tallying most in the measured data and corresponding to one another and via which with the aid of the technical, medical, diagnostic and person-specific information pertaining to the reference measurements of the measurement database and the comparison thereof with the information pertaining to the patient a diagnostic statement that is applicable to the patient with a certain probability can be inferred.

2. Evaluating system for obtaining diagnostic information from signals and data of medical sensor systems according to Point 1, **characterised in that** the measured data, which are measured and acquired in their totality as a measurement, of one or more sensor channels may represent both a continuous sequence of measured values over a certain period and one or more temporally limited segments of this continuous sequence of measured values.

3. Evaluating system for obtaining diagnostic information from signals and data of medical sensor systems according to Point 2, **characterised in that** the comparator may be realised as a computer program, as an electronic circuit or as a combination of both.

4. Evaluating system for obtaining diagnostic information from signals and data of medical sensor systems according to Point 1 to 3, **characterised in that** in addition to the comparison of the measured data of the patient and of the measurement database further person-related information known by the patient and stored in the measurement database can be drawn upon with a view to increasing the probability of the diagnostic statement relating to the patient.

5. Evaluating system for obtaining diagnostic information from signals and data of medical sensor systems according to Point 1 to 4, **characterised in that** the measurement database may contain both measured data and information, derived from the measured data of measurements both of healthy persons and of diseased persons, which reflects the biological diversity of the medical aspects of disease and of the patients as well as possible.

6. Evaluating system for obtaining diagnostic information from signals and data of medical sensor systems according to Point 1 to 5, **characterised in that** the measurement database can be extended by means of new reference data records.

7. Evaluating system for obtaining diagnostic information from signals and data of medical sensor systems according to Point 1 to 6, **characterised in that** the measurement database is stored either on an electronic storage medium directly in or at the evaluating system or is available via a local or worldwide data network and a spatial separation may exist between the recording system, the comparator and the reference database.

8. Evaluating system for obtaining diagnostic information from signals and data of medical sensor systems according to Point 1 to 7, **characterised in that** the signals and data may stem from various bioelectrical and/or biomagnetic and/or other biological sensor systems.

9. Evaluating system for obtaining diagnostic information from signals and data of medical sensor systems according to Point 1 to 8, **characterised in that** the signals recorded from a patient may be electrode potentials of an ECG recording system and/or signals of magnetic sensors of a single-channel or multichannel cardiological recording system.

## Revendications

1. Système d'évaluation pour l'acquisition d'informations diagnostiques à partir de signaux et de données de systèmes détecteurs à usage médical, lequel saisit simultanément par un ou plusieurs canaux sur un intervalle de temps déterminé les activités biologiques d'un patient, met en forme les données mesurées de chaque canal de détection et les met à disposition en totalité en tant que données mesurées relatives à une mesure, pour en tirer des informations diagnostiques, **caractérisé en ce que** les données mesurées issues d'un ou plusieurs canaux de détection relevées sur un patient, relatives à une mesure, sont comparées par un comparateur avec tout ou partie des données mesurées issues de canaux de détection comparables relatives à des mesures de référence, données qui sont stockées dans une ou plusieurs banques de données mesurées, de sorte que sont choisies dans les banques de données mesurées les mesures de référence qui possèdent par rapport à la mesure faite sur le patient aussi bien la plus grande similitude en ce qui concerne les données mesurées de canaux de détection comparables, que le plus grand nombre possible de canaux de détection mutuellement correspondants et coïncidant au mieux en ce qui concerne les données mesurées, et par l'intermédiaire desquelles il est possible de conclure à un énoncé diagnostique concernant le patient avec une certaine vraisemblance, à l'aide des informations techniques, médicales, diagnostiques et liées à la personne, relatives aux mesures de référence de la banque de données mesurées, et de la comparaison de ces informations avec celles relatives au patient.

2. Système d'évaluation pour l'acquisition d'informations diagnostiques à partir de signaux et de données de systèmes détecteurs à usage médical selon la revendication 1, **caractérisé en ce que** les données mesurées et saisies dans leur totalité en tant que mesure, d'un ou plusieurs canaux de détection, peuvent représenter aussi bien une suite continue de valeurs mesurées sur un intervalle de temps déterminé, qu'un ou plusieurs tronçons limités dans le temps issus de cette suite continue de valeurs mesurées.

3. Système d'évaluation pour l'acquisition d'informations diagnostiques à partir de signaux et de données de systèmes détecteurs à usage médical suivant la revendication 2, **caractérisé en ce que** le comparateur peut être réalisé aussi bien sous la forme d'un programme d'ordinateur, d'un circuit électronique ou d'une combinaison des deux.

4. Système d'évaluation pour l'acquisition d'informations diagnostiques à partir de signaux et de données de systèmes détecteurs à usage médical selon revendication 1 à 3, **caractérisé en ce qu'**à part la comparaison des données mesurées du patient et de la banque de données mesurées, on peut prendre en compte d'autres informations relatives à la personne, connues du patient et stockées dans la banque de données mesurées pour élever la vraisemblance de l'énoncé diagnostique concernant le patient.

5. Système d'évaluation pour l'acquisition d'informations diagnostiques à partir de signaux et de données de systèmes détecteurs à usage médical selon revendication 1 à 4, **caractérisé en ce que** la banque de données mesurée peut contenir aussi bien des données mesurées que des informations tirées de données mesurées, de mesures concernant aussi bien des personnes en bonne santé que des personnes malades, qui reflètent le mieux possible la multiplicité biologique des schémas pathologiques médicaux et des patients.

6. Système d'évaluation pour l'acquisition d'informations diagnostiques à partir de signaux et de données de systèmes détecteurs à usage médical selon revendication 1 à 5, **caractérisé en ce que** la banque de données mesurées peut être agrandie avec de nouveaux ensembles de données de référence.

7. Système d'évaluation pour l'acquisition d'informations diagnostiques à partir de signaux et de données de systèmes détecteurs à usage médical selon revendication 1 à 6, **caractérisé en ce que** la banque de données mesurées est ou bien stockée dans un moyen de stockage électronique directement dans le système d'évaluation ou à proximité de celui-ci, ou bien disponible par l'intermédiaire d'un réseau de données local ou mondial, et il peut y avoir une séparation spatiale entre le système de relevé, le comparateur et la banque de données de références.

8. Système d'évaluation pour l'acquisition d'informations diagnostiques à partir de signaux et de données de systèmes détecteurs à usage médical selon revendication 1 à 7, **caractérisé en ce que** les signaux et données peuvent provenir de différents systèmes capteurs bio-électriques et/ou bio-magnétiques et/ou autres systèmes capteurs biologiques.

9. Système d'évaluation pour l'acquisition d'informations diagnostiques à partir de signaux et de données de systèmes détecteurs à usage médical selon revendication 1 à 8, **caractérisé en ce que** les signaux relevés d'un malade peuvent être des potentiels d'électrodes d'un système de relevé d'électrocardiogramme et/ou des signaux de capteurs magnétiques d'un système de relevé cardiologique à un ou plusieurs canaux.
